# EUROPEAN PATENT APPLICATION

(11) **EP 2 497 467 A1**
(43) Date of publication of application: **12.09.2012**
(21) Application number: 10827738.5
(22) Date of filing: 05.11.2010
(51) Int. Cl.: A61K 31/06, A61K 31/429, A61K 31/4184, A61K 31/426, A61P 33/10

(54) **PHARMACEUTICAL COMBINATIONS, PHARMACEUTICAL COMPOSITIONS, MEDICAMENT AND METHOD FOR THE TREATMENT OF ANIMALS**

(30) Priority: 05.11.2009 BR PI0904365
(71) Applicant: Ouro Fino Participações E Empreendimentos S/A., 14025-230 BR Ribeirão Preto - SP (BR); NPA - Nucleo de Pesquisas Aplicadas Ltda., 14887-224 Jaboticabal SP (BR)
(72) Inventor: DOLIVAR CORACCI, Neto, CEP: 14160-040 (BR); FERNANDES FILHO, Nelson Henriques, CEP: 14887-226 (BR); DA SILVA SERCHELI, Ricardo, SP - CEP:14887-224 (BR)
(74) Representative: Eterno, Enrico
(86) International application number: PCT/BR2010/000361
(87) International publication number: WO 2011/054068

(57) **Abstract**

This invention relates to antihelminthic pharmaceutical combinations comprising iodinated aromatic compounds, such as disophenol or nitroxynil, with other pharmaceutical active ingredients targeting the treatment of animals. More specifically, this invention concerns pharmaceutical compositions comprising disophenol or nitroxynil with at least one other pharmaceutical antihelminthic active ingredient, selected from among benzimidazoles, thiabendazole, levamisole or tetramisole salts, avermectins or milbemycins and, even more preferably, levamisole salts selected from between levamisole chloride or phosphate.

This invention also relates to medications and a method for treating animals comprising such antihelminthic medications.

## Description

### Field of the Invention

This invention deals with new pharmaceutical combinations and their uses as antihelminthics comprised of iodinated aromatic compounds, with other active pharmaceutical ingredients for the treatment of animals. More specifically, the pharmaceutical combinations which are the object of this invention include disophenol or nitroxynil with at least one other active antihelminthic ingredient selected from among benzimidazole, thiabendazole compounds, levamisole or tetramisole salts, avermectins, or milbemycins. Preferably, the salts will be levamisole chloride and levamisole phosphate salts.

This invention also relates to compositions comprising such compounds, medications that contain them and methods for the treatment of animals.

### Background of the Invention

Helminthiasis is a disease of broad occurrence that affects different types of animals, causing financial damage to their breeders. Among others, animals that are particularly susceptible to these illnesses are sheep, goats and horses. Many antihelminthic agents, with different types of treatment effectiveness are currently used to combat this illness. For example, mebendazole is an antihelminthic with broad action, while levamisole acts specifically against nematodes in low concentrations.

Despite the efforts of researchers who have been trying for decades to increase the effectiveness of the treatment of helminthic infections in animals, resistance to anthelmintic compounds continues to grow, particularly in relation to the benzimidazole class compounds. One of the strategies to combat this resistance and to increase the effectiveness of current treatments is to avoid the indiscriminate use of anthelmintics or to alternate them with other drugs during treatment.

Another alternative form that is being used to increase treatment effectiveness is the development of new substances or simultaneous administration of different anthelmintic active ingredients. For example, as described by E.M. Bennet et al., J. Parasitology, 8 (1978) 463-466, the simultaneous administration of levamisole in combination with mebendazole or other benzimidazole substituted in position 2 increased antihelminthic activity of the benzimidazoles against tetrathyridium of Mesocestoides Corti in rats.

The simultaneous administration of anthelmintics, in search of a synergistic action between such compounds in order to obtain medications that presents greater antihelminthic activity than that presented by the compounds in isolation, is one of the most viable ways to reach these objectives. The association of active ingredients is broadly used to combat various pathogens, such as bacteria, protozoa, parasites, fungus, and protozoa.

Unfortunately, the strategies used to increase the effectiveness of the antihelminthic treatments in animals have not shown to be as effective as desired, which has caused the provision of pharmaceutical combinations that will have greater antihelminthic activity against pathogens to become of great interest and, above all, that may be easily obtained and that have preferably, a low cost of production.

The pharmaceutical combinations that this invention examines effectively solve the deficiencies of the aforementioned prior art.

### Purposes of the Invention

It is, therefore, the main objective of this invention to provide associations of pharmaceutical anthelmintic compounds that present greater efficacy when compared to those currently available on the market and described in the State of the Art.

It is also the objective of this invention to provide pharmaceutical compositions, medications, and treatment methods for animals, such as, among others, sheep, goats, and horses, comprising the use of the aforesaid associations of anthelmintic compounds. Description of the Invention

This invention concerns pharmaceutical combinations comprising iodinated aromatic compounds which present antihelminthic activity, specifically disophenol or nitroxynil, with at least one active pharmaceutical ingredient selected from among benzimidazoles, thiabendazoles, or levamisole or tetramisole salts, used for the treatment of worms. Alternately, said associations may include at least yet another active antihelminthic pharmaceutical ingredient.

In accordance with this invention, the iodinated aromatic compounds of the pharmaceutical combinations may be used in their neutral form or as their basic salts, such as sodium disophenol, monoethanolamine disophenolate, sodium nitroxinate or monoethanolamine nitroxinate.

Preferably, the pharmaceutical combinations of this invention comprise disophenol or nitroxynil compounds and levamisole salts.

The benzimidazoles used in the pharmaceutical combinations of this invention are presented in their neutral form or as salts and preferably are selected from the group formed by mebendazole, albendazole, albendazole sulfoxide, flubendazole, oxfendazole, fenbendazole, oxibendazole compounds or mixtures thereof. Levamisole or tetramisole are preferably used in the form of their salts prepared with hydrochloric or phosphoric acid, commonly called levamisole or tetramisole chloride or phosphate, respectively.

Disophenol (2,6-diiodo-4-nitrophenol) and nitroxynil (1-iodo-3-cyano-5-nitrofenol) are compounds used in the control of trematodes and certain nematode parasites. Thiabendazole is an antihelminthic active ingredient that acts as a fungicide and parasiticide. Levamisole (L-2,3,5,6-tetrahydro-6-phenyl-imidazo-[2,1,b]thiazole) is the levorotatory isomer of the racemate called tetramisole and it is an antiparasitic widely used in the control of intestinal nematodes and respiratory worms in warm blooded animals. Due to its basic character, it forms salts with organic and inorganic acids, depending on the pKa of the acid concerned. However, levamisole is normally manufactured and marketed as levamisole chloride or phosphate, which are the preferred forms for preparing the pharmaceutical combinations of this invention. When the compositions are prepared by combining the acid salts of levamisole or tetramisole and disophenol or nitroxynil in their neutral forms, a chemical interaction takes place between the compounds. Nevertheless, when the compositions are prepared by combining the basic salts of nitroxynil or disophenol and the acid salts of levamisole or tetramisole, such as for example, sodium and chloride nitroxinate or disophenolate or levamisole or tetramisole phosphate, the formation of disophenolate or nitroxinate of levamisole or similar compounds may occur when tetramisole is used, with the concomitant formation of the corresponding inorganic sodium salt. The application for patent number BR/PI0505716 of the same applicants, describes the levamisole salt prepared by combining levamisole with disophenol. Patent application number BR/PI0506279, also from the same applicants, describes pharmaceutical compositions prepared by combining this salt with other antihelminthic active ingredients, selected from between avermectins and milbemycins. These documents are herein attached as reference.

The results obtained in the treatment of animals using the combinations of this invention have been shown to be quite superior to the results obtained by administering two of these components in isolation. Surprisingly, it was thus observed that it is possible to obtain excellent results in the control of parasites when combinations of pharmaceuticals are prepared by simple combinations and application in the animals of nitroxynil or disophenol compounds with at least one other active antihelminthic ingredient selected from among benzimidazoles, thiabendazoles , or levamisole or tertramisole salts, especially for the treatment of worms. More specifically, these results were observed when pharmaceutical combinations were prepared comprising the disophenol compound and levamisole salts prepared with inorganic acids. In these cases, said pharmaceutical combinations, in accordance with this invention, were prepared in the form of pharmaceutical compositions including a pharmaceutically effective amount of each of the active ingredients, the dosages in final formulation being dependent upon the type of animal to be treated.

This invention also concerns pharmaceutical compositions with active ingredients including disophenol in quantities which range from 0.5% up to 50% by weight; nitroxynil in amounts ranging from 0.3% up to 30% by weight; levamisole in amounts ranging from 0.3% up to 30% by weight; and adjuvant pharmaceutically acceptable agents.

The active ingredients disophenol, nitroxynil, and levamisole may also be used in the form of their pharmaceutically acceptable salts. Such as pharmaceutically acceptable salts of disophenol, preferably sodium or monoethanolamine salts. Such as pharmaceutically acceptable salts of levamisole, preferably chlorhydrate or phosphate salts.

The pharmaceutical compositions in accordance with this invention may be prepared in several ways, such as grains, granules, powders, dissolved powders, solutions or suspensions, among other pharmaceutically acceptable agents. Preferably, the pharmaceutical compositions in accordance with this invention are prepared in their liquid form, in solution or suspension for oral or injectable use.

The adjuvant agents, or in other words, the vehicles and excipients, used in the pharmaceutical compositions in accordance with this invention, are generally solubilizing, suspensive, surfactants, preservatives, antioxidants, defoaming, solvent, co-solvent, flavoring, and sweetening agents and other pharmaceutically acceptable agents, such as those mentioned in several specialized publications, such as the Farmacopeia Americana, the Farmacopeia Brasileira, the Handbook of Pharmaceutical Excipients, among others.

Some non-restrictive examples are shown below of the compounds prepared in accordance with this invention. In these, the active ingredients disophenol, nitroxynil, and levamisole may be in the form of their pharmaceutically acceptable salts, as defined above.

Examples 1 and 2 - Suspensions for Oral Administration

### Example 1:

| COMPONENT | % WEIGHT | FUNCTION/OBSERVATIONS |
|---|---|---|
| Disophenol | 0,5-50 | Active Ingredient |
| Levamisole | 0.3-30 | Active Ingredient |
| Antimicrobial Preservative | 0.001-0.5 | Used in isolation or combined |
| Solvent | 1-80 | Solubilization of the components or vehicle of the formulation |
| Co-solvent | 1-60 | Assists with solubilization and wettability of the powdered components |
| Suspensive Agent | 0.01-50 | Increases viscosity and/or density |
| Surfactants | 0.05-15 | Reduces surface tension |
| Flavorings | 0.001-15 | Improves taste and smell |
| Defoaming Agent | 0.01-5 | Prevents the formation of foam |
| Antifloculant Agent | 0.1-2 | Balances the charges of the particles |

### Example 2:

| COMPONENT | % WEIGHT | FUNCTION/OBSERVATIONS |
|---|---|---|
| Nitroxynil | 0.3-30 | Active Ingredient |
| Levamisole | 0.3-30 | Active Ingredient |
| Antimicrobial Preservative | 0.001-0.5 | Used in isolation or combined |
| Solvent | 1-80 | Solubilization of the components or vehicle of the formulation |
| Co-solvent | 1-60 | Assists with solubilization and wettability of the powdered components |
| Suspensive Agent | 0.01-50 | Increases viscosity and/or density |
| Surfactants | 0.050-15 | Reduces surface tension |
| Flavorings | 0.001-15 | Improves taste and smell |
| Defoaming Agent | 0.01-5 | Prevents the formation of foam |
| Antifloculant Agent | 0.1-2 | Balances the charges of the particles |

Examples 3 and 4 - Solutions and/or Syrups for Oral Administration

### Example 3:

| COMPONENT | % WEIGHT | FUNCTION/OBSERVATIONS |
|---|---|---|
| Disophenol | 0.5-50 | Active Ingredient |
| Levamisole | 0.3-30 | Active Ingredient |
| Antimicrobial Preservative | 0.001-0.5 | Used in isolation or combined |
| Solvent | 1.00-80 | Solubilization of the components or vehicle of the formulation |
| Co-solvent | 1-60 | Assists solubilization or increases viscosity and wettability of the powdered components |
| Viscosity or sweetening agents | 0.01-50.00 | Stabilizing and sweetening agent |
| Surfactants | 0.05-15 | Reduces surface tension |
| Sweeteners | 0.001-15 | Improves flavor |
| Flavorings | 0.001-15 | Improves taste and smell |
| Defoaming Agent | 0.01-5 | Prevents foam formation |
| Antifloculant Agent | 0.1-2 | Balances the charges of the particles |

### Example 4:

| COMPONENT | % WEIGHT | FUNCTION/OBSERVATIONS |
|---|---|---|
| Nitroxynil | 0.3-30 | Active Ingredient |
| Levamisole | 0.3-30 | Active Ingredient |
| Antimicrobial Preservative | 0.001-0.5 | Used in isolation or combined |
| Solvent | 1-80 | Solubilization of the components or vehicle of the formulation |
| Co-solvent | 1-60 | Assists solubilization or increases viscosity and wettability of the powdered components |
| Viscosity or sweetening agents | 0.01-50 | Stabilizer and sweetener |
| Surfactants | 0.05-15 | Reduces surface tension |
| Sweeteners | 0.001-15 | Improves flavor |
| Flavorings | 0.001-15 | Improves taste and smell |
| Defoaming Agent | 0.01-5 | Prevents the formation of foam |
| Antifloculant Agent | 0.1-2 | Balances the charges of the particles |

Examples 5 and 6: Injectable or Parenteral Suspensions

### Example 5:

| COMPONENT | % WEIGHT | FUNCTION/OBSERVATIONS |
|---|---|---|
| Disophenol | 0.5-50 | Active Ingredient |
| Levamisole | 0.3-30 | Active Ingredient |
| Antimicrobial Preservative | 0.001-0.5 | Used in isolation or combined |
| Solvent | 1-80 | Solubilization of the components and/or formula filler |
| Co-solvent | 1-60 | Helps solubilization and/or increases density |
| Suspensive Agent | 0.01-50 | Increases viscosity and/or density |
| Surfactant | 0.05-15 | Reduces surface tension |
| Defoaming Agent | 0.01-5 | Prevents the formation of foam |
| Osmolarity Agent | 0.01-20 | Balances the osmolarity |

### Example 6:

| COMPONENT | % WEIGHT | FUNCTION/OBSERVATIONS |
|---|---|---|
| Nitroxynil | 0.3-30 | Active Ingredient |
| Levamisole | 0.3-30 | Active Ingredient |
| Antimicrobial Preservative | 0.001-0.5 | Used in isolation or combined |
| Solvent | 1-80 | Solubilization of the components and/or formula filler |
| Co-solvent | 1-60 | Helps solubilization and/or increases density |
| Suspensive Agent | 0.01-50 | Increases viscosity and/or density |
| Surfactant | 0.05-15 | Reduces surface tension |
| Defoaming Agent | 0.01-5 | Prevents the formation of foam |
| Osmolarity Agent | 0.01-20 | Balances the osmolarity |

### Examples 7 and 8: Injectable or Parenteral Solutions

### Example 7:

| COMPONENT | % WEIGHT | FUNCTION/OBSERVATIONS |
|---|---|---|
| Disophenol | 0.5-50 | Active Ingredient |
| Levamisole | 0.3-30 | Active Ingredient |
| Antimicrobial Preservative | 0.001-0.5 | Used in isolation or combined |
| Antioxidants | 0.001-5 | Prevents oxidation to reduce loss of active ingredient |
| Solvent | 1-80 | Solubilization of components |
| Co-solvent | 1-60 | Helps solubilization |
| Surfactant | 0.050-15 | Reduces surface tension |
| Defoaming Agent | 0.001-0.009 | Prevents the formation of foam |
| Osmolarity Agent | 0.01-20 | Balances the osmolarity |

### Example 8:

| COMPONENT | % WEIGHT | FUNCTION/OBSERVATIONS |
|---|---|---|
| Nitroxynil | 0.3-30 | Active Ingredient |
| Levamisole | 0.3-30 | Active Ingredient |
| Antimicrobial Preservative | 0.001-0.5 | Used in isolation or combined |
| Antioxidants | 0.001-5 | Prevents oxidation to reduce loss of active ingredient |
| Solvent | 1-80 | Solubilization of components |
| Co-solvent | 1-60 | Helps solubilization |
| Surfactant | 0.050-15 | Reduces surface tension |
| Defoaming Agent | 0.001-0.009 | Prevents the formation of foam |
| Osmolarity Agent | 0.01-20 | Balances the osmolarity |

Those versed in the art know choosing the various specific adjuvants to be used in each of the compounds of the above defined Examples 1-8. Therefore, in accordance with this invention, the following adjuvants are preferred:

- Suspensive and viscosity agents: one or more selected from the group consisting of gum tragacanth, gum arabic (acacia gum; acacia; talha gum), guar gum (polysaccharide of galactomannan; guar gum; guar flour; jaguar gum), sodium alginate (Algin), propylene glycol alginate, bentonite (taylorite; wilkinite; mineral soap; soap clay), kaolin (clay; China Clay), colloidal silica, aluminum and magnesium silicate, calcium carboxymethyl cellulose (carmellose calcium), sodium carboxymethyl cellulose (carmellose sodium), microcrystalline cellulose, dextrins, hydroxy ethylcellulose, hydroxy ethyl methyl cellulose, hydroxypropyl cellulose, hypromellose, methylcellulose, providone, agar (agar-agar), alginic acid, carragenin, ceratonia gum, gelatin, hectorite, polycarbophil, propylene glycol alginate, carbomer, pectin, chitosan, maltodextrin, polycarbophil, polyvinyl alcohol, saponite, sucrose and xanthan gum;

- Preservative or conservation agents: one or more selected from the group consisting of benzoic acid, benzyl alcohol, benzalkonium chloride, bronopol, chlorobutanol, chlorocresol, cresol, butylparaben, ethylparaben, methylparaben, propylparaben, imidurea, cetrimide, cetylpyridinium chloride, chlorhexidine, N-(3-chloroallyl)hexaminium chloride, chloroxylenol, hexetidine, sodium butyl paraben, sodium methyl paraben, sodium propyl paraben, isothiazolinone mixtures (methylchloroisothiazolinone and methylisothiazoninone), miripirio chloride, benzethonium chloride, boric acid, phenoxyethanol, phenylethyl alcohol, phenylmercury acetate, phenylmercury borate, phenylmercuric nitrate, potassium benzoate, potassium metabisulfite, potassium bisulfite, sodium bisulfite

sodium sulfite, sulfur dioxide, potassium sorbate, propionic acid, propylene glycol, sodium benzoate, sodium propionate, sorbic acid, thimerosal, glycerin, imidazolidinyl urea, o-phenyl phenol, β-phenylethyl alcohol and metacresol;

- Wetting and solubilizing/surfactant agents: one or more selected from the group consisting of Polysorbates (20, 21, 40, 60, 65, 80, 81, 85, 120), docusate sodium, lecithin, macrogol 15 hydroxystearate, poloxamer, polyoxyethylene alkyl esters (Cetomacrogol 1000, polyoxyl 6 cetostearyl ether, polyoxyl 20 cetostearyl ether, polyoxyl 25 cetostearyl ether, polyoxyl 2 cetyl ether, polyoxyl 10 cetyl ether, polyoxyl 20 cetyl ether, polyoxyl 4 lauryl ether, polyoxyl 9 lauryl ether, polyoxyl 23 lauryl ether, polyoxyl 2 oleyl ether, polyoxyl 10 oleyl ether, polyoxyl 20 oleyl ether, polyoxyl 2 stearyl ether, polyoxyl 10 stearyl ether, polyoxyl 21 stearyl ether, polyoxyl 100 stearyl ether), derivatives of polyoxyethylene castor oil (polyoxyl 5 Castor Oil, polyoxyl 9 Castor Oil, polyoxyl 15 Castor Oil, polyoxyl 35 Castor Oil, polyoxyl 40 Castor Oil, polyoxyl 60 Castor Oil, polyoxyl 100 Castor Oil, polyoxyl 200 Castor Oil, polyoxyl 40 hydrogenated Castor Oil, polyoxyl 60 hydrogenated Castor Oil, polyoxyl 100 hydrogenated Castor Oil, polyoxyl 200 hydrogenated Castor Oil), polyoxyethylene stearates (polyoxyl 2 stearate, polyoxyl 4 stearate, polyoxyl 6 stearate, polyoxyl 8 stearate, polyoxyl 12 stearate, polyoxyl 20 stearate, polyoxyl 30 stearate, polyoxyl 40 stearate, polyoxyl 50 stearate, polyoxyl 100 stearate, polyoxyl 150 stearate, polyoxyl 4 distearate, polyoxyl 8 distearate, polyoxyl 12 distearate, polyoxyl 32 distearate, polyoxyl 150 distearate), 2-pyrrolidone, sodium lauryl sulfate, sorbitan fatty acid esters (sorbitan monolaurate, sorbitan monooleate, sorbitan monopalmitate, sorbitan monostearate, sorbitan sesquioleate, sorbitan trioleate, sorbitan tristearate), stearic acid, benzalkonium chloride, benzyl benzoate, cetylpyridinium chloride and crospovidone;

- Co-solvents or wetting agents: one or more selected from the group consisting of propylene glycol, sodium lactate, glycerin, triacetin, xylitol, sorbitol, cyclomethicone, polydextrose, ammonium alginate, medium and long chain esters or triglycerides, polyethylene glycols, n-methyl-2-pyrrolidone and 2-pyrrolidone;

- Defoaming Agents: one or more selected from the group consisting of dimeticone, simeticone and, oleic alcohol;

- Solvents/Co-solvents: one or more selected from the group consisting of propylene carbonate, glycerin, propylene glycol, ethanol, almond oil, benzyl alcohol

benzyl benzoate, castor oil, corn oil, cottonseed oil, dibutyl phthalate, diethyl phthalate, dimethyl phthalate, dimethyl sulfoxide, dimethyl acetamide, ethyl acetate, ethyl lactate, ethyl oleate, glycofurol, isopropyl myristate, isopropyl palmitate, medium chain triglycerides, mineral oil, peanut oil, polyethylene glycol, sesame oil, soybean oil, sunflower seed oil and triacetin.

As a relevant aspect, this invention also takes into consideration pharmaceutical compositions comprising disophenol or nitroxynil and other active anthelmintic ingredients, selected from among abamectin, ivermectin, doramectin, eprinomectin, selamectin, moxidectin, milbemycin and milbemycin oxime. Moreover, as another additional relevant aspect, the pharmaceutical compositions of the invention comprise disophenol, levamisole or tetramisole salts selected from between levamisole or tertramisole chloride or phosphate and at least one of the above described compounds. Regardless of the compound of interest, those who are knowledgeable in the art must use a pharmacologically effective amount in the formulation of the active ingredients selected to be combined with disophenol or nitroxynil so that the results obtained will be satisfactory in the treatment of helminthiasis. Although there may be broad variations, depending upon the animal to be treated, the dosages of avermectins and milbemycins can range between 5 and 500 micrograms of the active ingredient per kilo of live weight of the animal, being that a dosage of around 200 micrograms provides satisfactory results in the control of worms.

Additionally, other pharmaceutical compositions may be prepared by combining disophenol with nitroxynil, or even by combining this mixture with effective amounts for the control of parasites with at least one active pharmaceutical ingredient selected from among levamisole or tetramisole salts, thiabendazole, mebendazole, albendazole, albendazole sulfoxide, flubendazole, oxfendazole, fenbendazole, oxibendazole, abamectin, ivermectin, doramectin, eprinomectin, selamectin, moxidectin, milbemycin and milbemycin oxime and preferably, levamisole in the form of their salts prepared with hydrochloric acid or phosphoric acid, in addition to adjuvant pharmaceutically acceptable agents.

Within another aspect of this invention, the pharmaceutical compositions may comprise disophenol, nitroxynil, or mixtures thereof with a pro-drug of at least one of the active antihelminthic ingredients of interest. The term pro-drug is here used to classify substances that have a chemical structure similar to the structure of the active ingredient and that are metabolically converted by the body of the animal in treatment into the end active ingredients of interest. In the case of benzimidazoles, one of the pro-drugs of interest and the carbamate known as netobimin, in spite of the similar compounds also included within the scope of this invention.

The pharmaceutical compositions which are the object of this invention may be obtained by any means desired to be selected by those who are knowledgeable in the art, depending on the industrial facilities available, but are preferably obtained by the simple combination of disophenol and/or nitroxynil with at least one additional active pharmaceutical ingredient selected from among the compounds previously mentioned and a pharmaceutically acceptable vehicle.

Additionally, this invention concerns antihelminthic medications for animals prepared from the above defined compositions and comprising an amount of disophenol and/or of nitroxynil sufficient to supply between 0.6 and 30 mg per kilo of the animal's weight, as well as an amount of levamisole sufficient to supply between 0.4 and 20 mg per kilo of the animal's weight, Preferably, the medications in accordance with this invention comprise an amount of disophenol or nitroxynil sufficient to supply between 3 and 10 mg per kilo of the animal's weight and an amount of levamisole sufficient to supply between 2 and 5 mg per kilo of the animal's weight. Nevertheless, it should be taken into consideration that an amount of disophenol or nitroxynil of around 5.5 mg per kilo of the animal's weight and an amount of levamisole of around 3.5 mg per kilo of the animal's weight provides satisfactory results for the control of worms.

When the compositions are prepared in liquid or paste form, they may be administered by oral and parenteral route, but preferably are used orally in amounts sufficient to achieve the objectives desired in the control of parasites in animals, as defined above.

When the compositions are intended for oral use, they should be prepared in a certain concentration so that it may be preferably administered in volumes of 5 to 15 ml for animals of small or medium size, such as hogs, sheep, goats, dogs and cats. When one wishes to treat large sized animals, such as horses and cattle, the volumes should be greater than 15 ml, but it is desirable for the medications to be prepared in such a way that the volumes of application not be greater than 20 ml. In any of these cases they may be administered, but they are not only limited to the treatment of hogs, birds, goats, sheep, horses, dogs, cats, and cattle, although the compositions are of particular interest for the treatment of goats, sheep, and cattle.

For oral administration in the form of tablets which are coated or not, the compositions of the invention may be combined with a pharmaceutically acceptable inert vehicle, such as lactose, amide, sacarose, glucose, derivatives of cellulose, magnesium stearate, dicalcium phosphate, calcium sulfate, mannitol, sorbitol, and similar vehicles. Moreover, when desired or necessary, agglutinants, lubricants, disintegrating agents and appropriate coloring agents may also be incorporated into the mixture. Appropriate agglutinants include amide, gelatin, natural sugars, such as glucose or beta-lactose, corn sweeteners, natural and synthetic gums, such as acacia, tragacanth or sodium alginate, carboxymethyl cellulose, polyethylene glycol, waxes and similar agents. Lubricants that may be used in these forms of dosages include sodium oleate, sodium stearate, magnesium stearate, sodium benzoate, sodium acetate, sodium chloride, and similar lubricants. Disintegrating agents include, without restriction, amide, derivatives of cellulose agar, bentonite, xanthan gum, and similar lubricants.

The compositions in accordance with this invention may also be administered in through liposome delivery systems or coupled with soluble or partially soluble polymers, as drug release vehicles.

For oral administration in liquid form, the components of the compositions of the invention may be combined with any oral vehicle that is inert and pharmaceutically acceptable, such as ethanol, glycerol, water, and similar vehicles. These types of liquid dosages for oral administration may contain colorants and flavorings to increase acceptance of the composition by the animal. In a general manner, water, an appropriate oil, saline solution, aqueous dextrose (glucose), sugar and glycol solutions, such as propylene glycol or polyethylene glycols, phosphate buffers are appropriate vehicles.

It should be noted that the medications produced by using the above described pharmaceutical compositions may be administered alone or even interspersed with the administration of medications intended for the treatment of other diseases in animals.

Yet another object of this invention is a treatment method for animals for the purpose of controlling helminthiasis, which is of great interest to breeders, since the pharmaceutical compositions of the invention are highly effective for this control. The method for the treatment of animals with the medications contained in the compositions of this invention are designed to kill internal parasites, such as for example, nematodes, cestodes, trematodes and flukes, in warm blooded animals, such as for example, cattle, hogs, goats, sheep, cats, dogs, horses, in dosages and for the purpose described herein for these combinations. The route of administration may be, for example, parenterally, i.e., intravenous, subcutaneous or intramuscular administration; by oral administration as a solution or suspension, in the form of tablets which are coated or not, powder, granules, elixirs, suspensions, syrup emulsions, bolus, capsules, optionally as controlled or delayed release formulations, or instantly dissolving tablets (flash type) or as a food additive. They may be administered through the skin using transdermal formulations of the pour-on type. The method of administration is preferably performed by means of a single effective dose, with the preferred form of administration being the oral route for small and medium sized animals, with the preferred form of administration being the oral or parenteral route for large animals such as cattle and horses.

As will be evident for those well-versed in the art, this invention provides various advantages in relation to the combinations, compositions and anthelmintic medicines described in the State of the Art, because they are highly effective in the control of parasites in warm-blooded animals.

## Claims

1. PHARMACEUTICAL COMBINATIONS, **characterized in that** they comprise the iodinated aromatic compounds disophenol or nitroxynil, and at least one pharmaceutical active ingredient selected from among the benzimidazoles, thiabendazoles or levamisole or tetramisole salts.

2. PHARMACEUTICAL COMBINATIONS, in accordance with Claim 1, **characterized by** the fact that they comprise disophenol or nitroxynil and the salts of levamisole.

3. PHARMACEUTICAL COMBINATIONS, in accordance with Claim 1, **characterized in that** they additionally comprise and at least one pharmaceutical antihelminthic active ingredient.

4. PHARMACEUTICAL COMBINATIONS, in accordance with Claim 1, **characterized by** the fact that the iodinated aromatic compounds disophenol and nitroxynil are used in their basic salt form, such as sodium disophenolate, monoethanolamine disophenolate, sodium nitroxinate, or monoethanolamine nitroxinate.

5. PHARMACEUTICAL COMBINATIONS, in accordance with Claim 1, **characterized by** the fact that the benzimidazole compounds are present in the neutral forms or as salts and selected from the group consisting of mebendazole, albendazole, albendazole sulfoxide, flubendazole, oxfendazole, fenbendazole, oxibendazole, or mixtures thereof.

6. PHARMACEUTICAL COMBINATIONS, in accordance with Claim 1, **characterized by** the fact that the levamisole or tetramisole compounds present in the form of their chloride or phosphate salts of levamisole or tetramisole, respectively.

7. PHARMACEUTICAL COMPOSITIONS, **characterized in that** they comprise as active ingredients, the disophenol compound in quantities ranging from 0.5% up to 50% by weight, or the nitroxynil compound or their pharmaceutically acceptable salts in amounts ranging from 0.3% up to 30% by weight, and the levamisole compound or their pharmaceutically acceptable salts in amounts ranging from 0.3% up to 30% by weight, in addition to adjuvant pharmaceutically acceptable agents.

8. PHARMACEUTICAL COMPOSITIONS, in accordance with Claim 7, **characterized by** the fact that the active ingredient is in the form of their pharmaceutically acceptable salts and the levamisole compound is in its free-base form.

9. PHARMACEUTICAL COMPOSITIONS, in accordance with Claim 7, **characterized in that** the active ingredient, levamisole is in one of the forms of its hydrochloric or phosphate salts.

10. PHARMACEUTICAL COMPOSITIONS, in accordance with Claim 8, **characterized in that** the active ingredient, disophenol is in one of the forms of its sodium or monoethanolamine salts.

11. PHARMACEUTICAL COMPOSITIONS, in accordance any of Claims 7 to 10, **characterized by** the fact that they are prepared in their liquid form, in solution or suspension for oral or injectable use, in the form of grains, granules, powder, wettable powders, suspensions or suspensions, among other pharmaceutically acceptable agents.

12. PHARMACEUTICAL COMPOSITIONS, in accordance with Claim 7, **characterized by** the fact that the adjuvant agents are generally solubilizing, suspensive, surfactant, preservative, antioxidant, defoaming, solvent, co-solvent, flavoring, sweetener, agents and other pharmaceutically acceptable agents.

13. PHARMACEUTICAL COMPOSITIONS, in accordance with Claim 7, **characterized by** the fact that they comprise mixtures of the disophenol compound or of the nitroxynil compound with levamisole salts selected from levamisole chloride, levamisole phosphate or mixtures thereof.

14. PHARMACEUTICAL COMPOSITIONS, in accordance with Claim 13, **characterized by** the fact that they also comprise at least one other pharmaceutical antihelminthic active ingredient.

15. PHARMACEUTICAL COMPOSITIONS, in accordance with Claim 7, **characterized in that** they comprise mixtures of the disophenol compound or of the nitroxynil compound with tetramisole salts selected from tetramisole chloride, tetramisole phosphate or mixtures thereof.

16. PHARMACEUTICAL COMPOSITIONS, in accordance with Claim 7, **characterized in that** they comprise mixtures of the disophenol compound or of the nitroxynil compound with tetramisole salts selected from tetramisole chloride, tetramisole phosphate or mixtures thereof.

17. PHARMACEUTICAL COMPOSITIONS, in accordance with Claim 7, **characterized in that** comprising concomitantly disophenol and nitroxynil combined with pharmacologically effective amounts for the control of parasites with at least one active ingredient selected from among the salts of levamisole or tetramisole, thiabendazole, mebendazole, albendazole, albendazole sulfoxide, flubendazole, oxfendazole, fenbendazole, oxibendazole, abamectin, ivermectin, doramectin, eprinomectin, selamectin, moxidectin, milbemycin and milbemycin oxime, preferably, levamisole in the form of their salts prepared with hydrochloric acid or phosphoric acid, in addition to adjuvant pharmaceutically acceptable agents.

18. PHARMACEUTICAL COMPOSITIONS, in accordance with Claim 7, **characterized in that** they additionally comprise a pharmaceutical antihelminthic active ingredient selected from thiabendazole, mebendazole, albendazole, albendazole sulfoxide, flubendazole, oxfendazole, fenbendazole, oxibendazole, abamectin, ivermectin, doramectin, eprinomectin, selamectin, moxidectin, milbemycin or milbemycin oxime or their corresponding pro-drugs.

19. PHARMACEUTICAL COMPOSITIONS, in accordance with Claim 18, **characterized by** the fact that the pro-drug is a carbamate called netobimin.

20. MEDICATION, for the antihelminthic treatment of animals, **characterized in that** they are manufactured from the compositions defined in Claims 7 to 19 above, to provide an amount of disophenol and/or nitroxynil sufficient to supply between 0.6 and 30 mg per kilo of the animal's weight, as well as an amount of levamisole sufficient to supply between 0.4 and 20 mg per kilo of the animal's weight.

21. MEDICATION, in accordance with Claim 20, **characterized by** the fact that it comprises an amount of disophenol and/or nitroxynil sufficient to supply between 3 and 10 mg per kilo of the animal's weight and an amount of levamisole sufficient to supply between 2 and 5 mg per kilo of the animal's weight.

22. MEDICATION, in accordance with Claim 21, **characterized by** the fact that it comprises an amount of disophenol and/or nitroxynil sufficient to supply around 5.5 mg per kilo of the animal's weight and an amount of levamisole of around 3.5 mg per kilo of the animal's weight.

23. MEDICATION, in accordance any of Claims 20 to 22, **characterized by** the fact that it is produced in solid or in liquid form, through combining them with vehicles and other pharmaceutically acceptable components.

24. METHOD FOR THE ANTIHELMINTHIC TREATMENT OF ANIMALS, **characterized by** the fact that it includes the administration of a medication as defined in Claims 20 to 23 above, comprised by an effective amount of a pharmaceutically acceptable form for the treatment of helminthiasis in animals, to provide an amount of disophenol and/or nitroxynil sufficient to supply between 0.6 and 30 mg per kilo of the animal's weight, as well as an amount of levamisole sufficient to supply between 0.4 and 20 mg per kilo of the animal's weight.

25. METHOD FOR THE ANTIHELMINTHIC TREATMENT OF ANIMALS, in accordance with Claim 24, **characterized by** the fact that it includes forms of administration selected between the parenteral route, such as intravenously, subcutaneously or intramuscularly; oral route such as suspensions or suspensions, tablets, bolus, capsules or as a food additive or by transdermal administration of the pour-on type.
